# EUROPÄISCHE PATENTSCHRIFT

(11) **EP 3 423 826 B1**
(45) Veröffentlichungstag und Bekanntmachung des Hinweises auf die Patenterteilung: **21.10.2020**
(21) Anmeldenummer: 17709615.3
(22) Anmeldetag: 01.03.2017
(51) Int. Cl.: G01N 33/487, G01N 27/02

(54) **VERFAHREN ZUR KALIBRATION VON IMPEDANZSPEKTROSKOPISCHEN BIOMASSESENSOREN UND VERWENDUNG EINER SUSPENSION ZUR DURCHFÜHRUNG EINES SOLCHEN VERFAHRENS**
METHOD FOR CALIBRATING BIOMASS SENSORS OPERATING WITH IMPEDANCE SPECTROSCOPY AND USE OF A SUSPENSION FOR CARRYING OUT SUCH A METHOD
PROCÉDÉ D'ÉTALONNAGE DE CAPTEURS DE BIOMASSE PAR SPECTROSCOPIE D'IMPÉDANCE ET UTILISATION D'UNE SUSPENSION POUR EXÉCUTER UN PROCÉDÉ DE CE TYPE

(30) Priorität: 04.03.2016 DE 102016203576
(43) Veröffentlichungstag der Anmeldung: 09.01.2019
(73) Patentinhaber: Hamilton Bonaduz AG, 7402 Bonaduz (CH)
(72) Erfinder: FRANK, Marlene, 7402 Bonaduz (CH); ARQUINT, Philipp, 7402 Bonaduz (CH); GAUPP, Theo, 7204 Untervaz (CH)
(74) Vertreter: Ruttensperger Lachnit Trossin Gomoll
(86) Internationale Anmeldenummer: PCT/EP2017/054751
(87) Internationale Veröffentlichungsnummer: WO 2017/149005

(56) Entgegenhaltungen:
- EP-A2- 1 085 316
- US-A1- 2008 312 843
- US-B2- 7 930 110
- DABROS M ET AL: "Cole-Cole, linear and multivariate modeling of capacitance data for on-line monitoring of biomass", BIOPROCESS AND BIOSYSTEMS ENGINEERING, SPRINGER, DE, Bd. 32, Nr. 2, 1. Februar 2009 (2009-02-01), Seiten 161-173, XP002574589, ISSN: 1615-7591, DOI: 10.1007/S00449-008-0234-4 [gefunden am 2008-06-11]
- ZHU ZHEN ET AL: "Real-time monitoring of immobilized single yeast cells through multifrequency electrical impedance spectroscopy", ANALYTICAL AND BIOANALYTICAL CHEMISTRY, SPRINGER, DE, Bd. 406, Nr. 27, 11. Juli 2014 (2014-07-11), Seiten 7015-7025, XP035407679, ISSN: 1618-2642, DOI: 10.1007/S00216-014-7955-9 [gefunden am 2014-07-11]

## Beschreibung

Die vorliegende Erfindung betrifft ein Verfahren zur Kalibration von impedanzspektroskopischen Biomassesensoren gemäß dem Oberbegriff von Anspruch 1 und betrifft weiter die Verwendung einer Suspension für ein solches Kalibrationsverfahren.

Elektrische Impedanzspektroskopieverfahren werden als Messverfahren zur zerstörungsfreien in-situ- und in-vivo-Bestimmung von frequenzabhängigen passiven elektrischen Eigenschaften von biologischen Materialien verwendet. Ein solches biologisches Material kann beispielsweise eine nachfolgend als "Biomasse" bezeichnete Substanz aus einer Flüssigkeit und darin aufgenommenen biologischen Zellen sein, wie sie etwa aus Zellkulturbehältern erhalten wird.

Die oben genannten frequenzabhängigen passiv-elektrischen Eigenschaften der Biomasse können Auskunft unter anderem über die Lebendzellzahl und die Zellvitalität geben. Daneben können noch weitere Eigenschaften der Biomasse durch die frequenzabhängigen passiv-elektrischen Eigenschaften ermittelt werden, jedoch weist die Bestimmung der Anzahl an lebenden Zellen und der Zellvitalität die größte Bedeutung in biotechnologischen Anwendungen auf.

Ganz grundsätzlich und grob skizziert erfolgt die impedanzspektroskopische Erfassung von Information über die Menge oder/und Größe von lebenden Zellen in der Biomasse wie folgt: An eine Suspension von in einer Trägerflüssigkeit aufgenommenen biologischen Zellen wird ein elektrisches Feld angelegt, dessen Feldrichtung sich periodisch mit einer vorgegebenen Frequenz ändert. Abhängig von der gewählten Änderungsfrequenz wirken an der Biomasse unterschiedliche Polarisationsmechanismen. Daher sind die passiv-elektrischen Eigenschaften von Biomasse im Gegensatz zu rein ionischen Lösungen frequenzabhängig und bilden typische sogenannte Dispersionen aus. Befinden sich in der Biomasse lebende Zellen, entstehen zusätzliche Effekte. Die lebenden Zellen stellen ein Wanderungshindernis für frei bewegliche Ionen dar, sodass die Zellen abhängig von ihrer Querschnittsfläche den Widerstand der Biomasse erhöhen und folglich deren Leitfähigkeit verringern. Auch die im Inneren einer Zelle der Biomasse vorhandenen Ionen werden von dem elektrischen Feld erfasst. An der elektrisch schlecht leitenden Zellmembran tritt eine Ladungstrennung auf, die zu einer Polarisation an der Membran führt. Die Polarisation tritt als elektrische Doppelschicht auf. In diesem Fall fließt der durch das elektrische Feld bewirkte Strom fast ausschließlich durch die extrazelluläre Trägersubstanz der Biomasse. Die Polarisierung spiegelt sich daher in der Erhöhung der messbaren Kapazität und folglich der Permittivität zwischen den felderzeugenden Elektroden wieder.

Eine sogenannte "a-Dispersion" tritt im Millihertz- bis Kilohertz-Frequenzbereich auf und wird durch die Bewegung und Anlagerung von Ladungsträgern auf der Zelloberfläche hervorgerufen. Weiter beeinflussen aktive Zellmembraneffekte und aktive ionische Membrankanäle die α-Dispersion.

In einem Frequenzbereich von einigen Kilohertz bis Hundert Megahertz tritt eine sogenannte β-Dispersion auf, die auf den kapazitiven Eigenschaften der Zellmembranen, den intrazellulären Organellen und den Membranstrukturen selbst beruht. Die Fette und Proteine in den Zellmembranen bilden eine hochohmige Struktur. Die Membranstrukturen führen zu sogenannter Grenzflächenpolarisation.

Bei noch höheren Frequenzen im Bereich von ca. 0,1 bis 100 GHz tritt die sogenannte γ-Dispersion auf, die durch dipolare Mechanismen von polaren Medien, wie beispielsweise Wasser und Proteinen, hervorgerufen wird. Im elektrischen Feld richten sich bei hohen Frequenzen große Moleküle, wie z. B. Proteine, mit Dipolmoment aus.

Für die im Verfahren der vorliegenden Anmeldung besonders interessierende Erfassung von Information über die Menge oder/und Größe von lebenden Zellen in einer Biomasse ist vor allem das β-Dispersionsgebiet von Interesse. Zur weiterführenden Information über die Dispersionsgebiete von Biomassen und die zu ihrer Darstellung verwendeten Kurven einer frequenzabhängigen Permittivität oder Kapazität wird verwiesen auf H. P. Schwan: "The practical success of impedance techniques from an historical perspecitve", in: Proceedings of the X. International Conference on Electrical Bio-Impedance, Barcelona (1998), Seiten 3 bis 16, sowie auf H. P. Schwan: "Electrical properties of tissue and cell suspensions", in: Advances in Biological and Medical Physics, Vol. 5 (1957), Seiten 147 bis 205.

Ein bekanntes Verfahren zur impedanzspektroskopischen Erfassung von Information über die Menge oder/und Größe von lebenden Zellen in einer Biomasse mittels eines elektrischen Feldes mit sich periodisch ändernder Feldrichtung ist aus der US 7 930 110 B2 bekannt. Ein für eine derartige Messung einsetzbarer Biomassesensor ist aus der US 6 596 507 B2 bekannt. Auch ein von der Anmelderin unter dem Handelsnamen "Incyte" vertriebener Sensor ist für das oben beschriebene Messverfahren grundsätzlich geeignet.

Anwender der oben beschriebenen impedanzspektroskopischen Messtechnik - "Messtechnik" ist dabei ganz allgemein sowohl hinsichtlich des Verfahrensaspekts als auch hinsichtlich der verwendeten Sensoren zu verstehen - stehen dabei vor der Situation, dass sie in der Regel auf die Funktionstüchtigkeit des Sensors vertrauen müssen, da sie üblicherweise keine Möglichkeit haben, den Sensor in einem für den späteren Messbetrieb aussagekräftigen Kalibrationsbetrieb hinsichtlich seiner Funktionsfähigkeit zu überprüfen.

Aus Dabros, Michal; Currie, David J. und Marison, lan W.: "Cole-Cole, linear and multivariate modeling of capacitance data for on-line monitoring of biomass" in Bioprocess Biosyst Eng (2009), Springer-Verlag, Seiten 161 bis 173, ist ein Verfahren bekannt zur Kalibration von Biomassesensoren, welche zur Erfassung von Information über die Menge oder/und Größe von lebenden Zellen in einer Biomasse mittels eines elektrischen Feldes mit sich periodisch ändernder Feldrichtung ausgebildet sind. Das bekannte Verfahren umfasst die folgenden Schritte:
- Bereitstellen einer Kalibrationssuspension mit in einer Trägerflüssigkeit aufgenommenen Zellen als Festkörperpartikel,
- Erzeugen eines auf die Kalibrationssuspension einwirkenden elektrischen Feldes mit sich periodisch ändernder Feldrichtung,
- Erfassen von wenigstens je einem eine Permittivität der Kalibrationssuspension repräsentierenden Permittivitätswert bei wenigstens zwei elektrischen Feldern unterschiedlicher Frequenz der Feldrichtungsänderung, und
- Ermitteln eines einen Unterschied der erfassten Permittivitätswerte repräsentierenden Unterschiedswertes.

Aus der EP 1 085 316 A2 ist es bekannt, Änderungen von Zell-Parameter oder -zuständen durch Impedanzmessungen einer Zellsuspension bei einer Reihe von unterschiedlichen Frequenzen durchzuführen. Dabei werden diese Messungen dazu benutzt, jegliche Veränderung in der frequenz-abhängigen Impedanzcharakteristik zu ermitteln. Eine solche Veränderung zeigt andere Änderungen als Änderungen der Zellkonzentration oder der Leitfähigkeit des Mediums an, in welchem die Zellen suspendiert sind.

Aus Zhu, Zhen et al.: "Real-tie monitoring of immobilized single yeast cells through multifrequency electrical impedance spectroscopy" in Anal Bioanal Chem (2014), Springer-Verlag, Seiten 7015 bis 7025, ist es bekannt, einzelne Zellen durch Multifrequenz-Impedanzspektroskopie hinsichtlich Zellwachstum und potenzielle Zellbewegung in der Zellfalle der Überwachungseinrichtung zu überwachen. Zur Charakterisierung der Vorrichtung wird eine Suspension mit Polystyrol-Teilchen verwendet. Z

Aufgabe der vorliegenden Erfindung ist es, eine technische Lehre anzugeben, die eine aussagekräftige Kalibration von impedanzspektroskopischen Biomassesensoren zur Erfassung von Information über die Menge oder/und Größe von lebenden Zellen in einer Biomasse mittels eines elektrischen Feldes mit sich periodisch ändernder Feldrichtung gestattet.

Diese Aufgabe wird erfindungsgemäß gelöst durch ein Verfahren zur Kalibration von impedanzspektroskopischen Biomassesensoren, welche zur Erfassung von Information über die Menge oder/und Größe von lebenden Zellen in einer Biomasse mittels eines elektrischen Feldes mit sich periodisch ändernder Feldrichtung ausgebildet sind, mit allen Merkmalen des Anspruchs 1.

Es hat sich herausgestellt, dass die oben bezeichnete Kalibrationssuspension, welche eine elektrisch leitfähige, viskos-fließfähige oder viskoelastische Trägersubstanz und darin aufgenommene elektrisch leitfähige Festkörperpartikel oder/und Halbleiter-Festkörperpartikel umfasst, im Zusammenwirken mit den für das eingangs genannte impedanzspektroskopische messtechnische Verfahren zur Erfassung von Information über die Menge oder/und Größe von lebenden Zellen ausgebildeten Biomassesensoren auf das sich periodisch ändernde elektrische Feld eine ähnliche Antwort liefert wie auch die Biomasse selbst. Daher kann aus der Antwort der Kalibrationssuspension auf das sich hinsichtlich der Feldrichtung periodisch ändernde elektrische Feld auf die Funktionstüchtigkeit des verwendeten Biomassesensors geschlossen werden.

Deshalb wird im Rahmen des erfindungsgemäßen Kalibrationsverfahrens ein auf die Kalibrationssuspension einwirkendes elektrisches Feld mit sich periodisch ändernder Feldrichtung erzeugt, das im Wesentlichen dem auf die Biomasse bei einer realen Informationserfassung einwirkenden elektrischen Feld ähnlich oder gleich ist.

Wie eingangs im Rahmen der Darstellung der technischen Zusammenhänge von impedanzspektroskopischen Erfassungsverfahren dargelegt, sind die hier interessierenden passiv-elektrischen Eigenschaften der Biomasse und damit ebenso der Kalibrationssuspension frequenzabhängig, sodass zur Kalibration des wenigstens einen Biomassesensors bei wenigstens zwei elektrischen Feldern unterschiedlicher Frequenz der Feldrichtungsänderung jeweils ein eine Permittivität der Kalibrationssuspension repräsentierender Permittivitätswert erfasst werden kann.

Dabei sei klargestellt, dass dies nicht notwendigerweise bedeutet, dass die Permittivität unmittelbar erfasst werden muss. Es genügt - wie gesagt - die Erfassung eines Wertes aus, der die Permittivität der Kalibrationssuspension repräsentiert. Dies kann beispielsweise eine Kapazität sein, die in der Regel proportional zur Permittivität ist.

Es wird bei wenigstens zwei unterschiedlichen Frequenzen der Feldrichtungsänderung der Permittivitätswert erfasst und ein Unterschiedswert ermittelt welcher einen Unterschied der erfassten Permittivitätswerte repräsentiert. Dies kann durch einfache Subtraktion der bei den jeweiligen Feldrichtungsänderungsfrequenzen erfassten Permittivitätswerte geschehen.

Da die Kalibrationssuspension mit vordefinierten chemischen und physikalischen Eigenschaften hergestellt werden kann, kann für sie eine Mehrzahl von Bezugs-Permittivitätswerten oder von Bezugswert-Unterschiedswerten vorab, beispielsweise durch Referenzmessungen im Labor, bestimmt werden. Ein solcher Bezugswert kann dann für einen Vergleich mit dem erfassten Unterschiedswert herangezogen werden. Anhand des dann ermittelten Vergleichsergebnisses ist der Benutzer des wenigstens einen Biomassesensors in der Lage, die Funktionstüchtigkeit des gemäß dem oben genannten Verfahren kalibrierten Biomassesensors zu beurteilen.

Auch die Beurteilung des Vergleichs des Unterschiedswertes mit dem der Kalibrationssuspension zugeordneten Bezugswert kann als vorteilhafte Weiterbildung von dem Kalibrationsverfahren umfasst sein. Beispielsweise kann dies vollautomatisiert in einer Auswerteeinrichtung erfolgen, die mit dem wenigstens einen Biomassesensor signalübertragungsmäßig verbunden ist. Die Auswerteeinrichtung kann über einen Datenspeicher verfügen, in dem wenigstens der eine zugeordnete Bezugswert oder eine Mehrzahl an der Kalibrationssuspension zugeordneten Bezugswerten hinterlegt sein kann. Beispielsweise kann in dem Datenspeicher ein Kennfeld hinterlegt sein, in welchem für eine oder mehrere Kalibrationssuspensionen ein Zusammenhang zwischen Permittivitätswerten und Feldrichtungsänderungsfrequenzen angegeben ist. Anstelle eines Kennfelds kann auch ein funktionaler Zusammenhang hinterlegt sein, welcher es der Auswerteeinrichtung ermöglicht, einen Bezugswert abhängig von den beim Kalibrationsverfahren verwendeten Feldrichtungsänderungsfrequenzen zu errechnen.

Weiter kann das Verfahren eine Ausgabe des Beurteilungsergebnisses an einer Ausgabeeinrichtung, wie etwa einem Bildschirm oder einem Drucker, umfassen.

Der Vorteil des erfindungsgemäßen Kalibrationsverfahrens liegt vor allem darin, dass mit einer Kalibrationssuspension, die selbst keine Biomasse, also lebende oder tote Zellen oder Zellbestandteile, enthält und daher kein Kontaminationsrisiko für tatsächliche Biomasse darstellt, das Kalibrationsverfahren so ablaufen kann, wie auch das impedanzspektroskopische Biomasse-Messverfahren abläuft. Folglich kann der wenigstens eine Biomassesensor wenigstens mit einem Abschnitt, insbesondere einem messtechnisch wirksamen Abschnitt, vor oder/und bevorzugt während des Erzeugens des sich in seiner Feldrichtung periodisch ändernden Feldes die Kalibrationssuspension kontaktieren. Dadurch, dass so das Kalibrationsverfahren dem realen impedanzspektroskopischen Biomasse-Messverfahren bis auf die Suspension als Messobjekt entspricht, können äußerst genaue Kalibrationsergebnisse erzielt werden. Darüber hinaus braucht für einen wenigstens teilautomatisierten Messaufbau kein gesonderter Kalibrationsverfahrensablauf programmiert werden, sondern es kann der Ablauf des realen Messverfahrens auch zur Kalibration genutzt werden.

Führt man das Kalibrationsverfahren ohne Benetzung des Biomassesensors mit der Kalibrationssuspension aus, werden zwar auch Ergebnisse erzielt, diese schwanken jedoch trotz im Wesentlichen gleicher Versuchsbedingungen erheblich und sind daher in ihrer Aussagekraft gegenüber dem den Sensor benetzenden Kalibrieren reduziert.

Zur Erzielung möglichst präziser Kalibrationsergebnisse ist daher bei einer bevorzugten Weiterbildung der Erfindung vorgesehen, dass das Kontaktieren der Kalibrationssuspension mit wenigstens einem Abschnitt des Biomassesensors ein Benetzen wenigstens einer Sensorelektrode, vorzugsweise aller Sensorelektroden, des Biomassesensors mit Kalibrationssuspension umfasst. Sofern der Biomassesensor mehr als nur eine Sensorelektrode aufweist, werden vorzugsweise alle Sensorelektroden des Biomassesensors mit Kalibrationssuspension benetzt, die bei einer impedanzspektroskopischen Erfassung der Biomasse von der Biomasse benetzt wären.

Um wiederholbare und daher zuverlässige und nachprüfbare Kalibrationsergebnisse bereitzustellen, ist es bevorzugt, wenn das Kalibrationsverfahren einen Schritt der Homogenisierung der Kalibrationssuspension umfasst. Die in der Trägersubstanz aufgenommenen Festkörperpartikel können sich - abhängig von der Viskosität der Trägersubstanz - mit fortschreitender Zeit absetzen und so die Kalibrationssuspension in ihrem Ansprechverhalten auf das äußere elektrische Feld verändern. Um einem derartigen Sedimentierungstrend entgegenzuwirken, kann der Schritt der Homogenisierung ausgeführt werden. Diese Homogenisierung kann durch Rühren oder/und Schütteln der Kalibrationssuspension erfolgen. Die Homogenisierung kann vor dem Erfassen der Permittivitätswerte erfolgen, bevorzugt unmittelbar davor, um die Erfassung an einer möglichst homogenen Kalibrationssuspension zu gestatten.

Die Dauer der Kalibration zur Bestimmung der Permittivitätswerte liegt in einem Bereich von 1 bis 600 Sekunden, bevorzugt 1 bis 300 Sekunden und besonders bevorzugt in einem Bereich von 1 bis 60 Sekunden. Über diese Zeitdauer hinweg sollte möglichst keine Sedimentierung stattfinden.

Für die Kalibration der impedanzspektroskopischen Biomassesensoren ist vor allem der eingangs genannte β-Dispersionsbereich wegen seiner Aussagekraft interessant. Daher ist es für das erfindungsgemäße Kalibrationsverfahren bevorzugt, dass die Permittivitätswerte bei Frequenzen von nicht weniger als 50 kHz, vorzugsweise nicht weniger als 110 kHz und besonders bevorzugt von nicht weniger als 250 kHz und von nicht mehr als 110 MHz, vorzugsweise von nicht mehr als 50 MHz, besonders bevorzugt von nicht mehr als 30 MHz, erfasst werden, die genannten Grenzwerte jeweils eingeschlossen. Dadurch wird das Kalibrationsverfahren bei Frequenzen ausgeführt, die jenen von β-Dispersionsbereichen einer real zu erfassenden Biomasse entsprechen.

Nicht nur kann sich ein Benutzer mit dem Kalibrationsverfahren einen Eindruck über die Funktionstüchtigkeit des wenigstens einen von ihm verwendeten impedanzspektroskopischen Biomassesensors verschaffen, sondern er kann das Kalibrationsergebnis quantitativ auch dazu nutzen, einen etwaigen Erfassungsfehler des wenigstens einen Biomassesensors zu korrigieren. Auch dies kann automatisiert in einer Steuereinrichtung der den wenigstens einen Biomassesensor aufweisenden Messanordnung oder in der oben genannten signalübertragungsmäßig mit dem wenigstens einen Biomassesensor zusammenhängenden Auswerteeinrichtung erfolgen. Daher kann das Kalibrationsverfahren gemäß einer vorteilhaften Weiterbildung ein Justieren des Biomassesensors oder einer mit ihm signaltübertragungsmäßig gekoppelten Datenverarbeitungsvorrichtung nach Maßgabe des Ergebnisses des Vergleichs des Unterschiedswertes mit dem Bezugswert umfassen. Somit kann ein Biomassesensor, der, beispielsweise aufgrund eines Driftfehlers und dgl., ein eigentlich unrichtiges Erfassungsergebnis von Permittivitätswerten liefern würde, auf Grundlage des Vergleichs des Unterschiedswerts mit dem Bezugswert derart justiert werden, dass die von ihm erfassten Permittivitätswerte den unter den jeweiligen Messumständen tatsächlich zu erwartenden Permittivitätswerten entsprechen. Vor allem - aber nicht nur - die Möglichkeit, den Biomassesensor abhängig vom Vergleichsergebnis des Unterschiedswerts mit dem Bezugswert zu justieren, schafft die Möglichkeit, sicherzustellen, dass in einem impedanzspektroskopischen Erfassungsverfahren, wie es eingangs beschrieben wurde, stets ein korrektes Erfassungsergebnis erzielt wird, solange der Biomassesensor justierbar ist bzw. einen justierbaren Fehler aufweist. Hierzu kann das Kalibrationsverfahren, wie es oben beschrieben und weitergebildet ist, vor einem an Biomasse durchgeführten realen Messverfahren oder vor einer Reihe von realen Messverfahren durchgeführt werden, um sicherzustellen, dass die Erfassungsergebnisse des wenigstens einen nach dem Kalibrationsverfahren ausgeführten Messverfahren korrekt sind.

Daher betrifft die vorliegende Erfindung auch ein Verfahren zur impedanzspektroskopischen Erfassung von Information über die Menge oder/und Größe von lebenden Zellen in einer Biomasse, umfassend
- das Kalibrationsverfahren, wie es oben beschrieben und weitergebildet ist,
- dem nachfolgend: Reinigen des Biomassesensors,
- dem nachfolgend: impedanzspektroskopische Erfassung der Information über die Menge oder/und Größe von lebenden Zellen in der Biomasse mittels des Biomassesenors und einem von diesem erzeugten elektrischen Feld mit sich periodisch ändernder Feldrichtung.

Für hochsensible impedanzspektroskopische Erfassungsanwendungen kann sogar vorgesehen sein, ein Kalibrationsverfahren nicht nur vor der impedanzspektroskopischen Erfassung mit der Biomasse als Messobjekt, sondern auch nach dieser Erfassung ein Kalibrationsverfahren durchzuführen, wie es oben beschrieben und weitergebildet ist. Dadurch kann beispielsweise für Dokumentations- und Zertifikationszwecke sichergestellt und nachgewiesen werden, dass der Biomassesensor vor und nach seiner realen Erfassungsverwendung an der Biomasse korrekt funktioniert hat und somit mit an Sicherheit grenzender Wahrscheinlichkeit auch zu erwarten ist, dass er während der impedanzspektroskopischen Erfassung korrekt funktioniert hat.

Der Kern des Kalibrationsverfahrens ist der Austausch der Biomasse als Messobjekt durch die Kalibrationssuspension, die frei von biologischen Bestandteilen in definierter Zusammensetzung herstellbar ist und somit eine Kreuzkontamination von Biomasse mit anderer Biomasse ausschließt. Daher betrifft die vorliegende Erfindung auch die Verwendung einer von biologischen Bestandteilen freien Suspension, umfassend eine elektrisch leitfähige viskos-fließfähige oder viskoelastische Trägersubstanz und darin aufgenommene elektrisch leitfähige Festkörperpartikel oder/und Halbleiter-Festkörperpartikel, zur Kalibration von impedanzspektroskopischen Biomassesensoren, welche zur Erfassung von Information über die Menge oder/und Größe von lebenden Zellen in einer Biomasse mittels eines elektrischen Feldes mit sich periodisch ändernder Feldrichtung ausgebildet sind.

Um gewährleisten zu können, dass die Festkörperpartikel in der Kalibrationssuspension wenigstens für die Dauer des Kalibrationsverfahrens eine lediglich vernachlässigbare Sedimentierungstendenz aufweisen, weist die Kalibrationssuspension bevorzugt eine Trägersubstanz mit einer dynamischen Viskosität von nicht weniger als 1 Pas, bevorzugt von nicht weniger als 50 Pas, und von nicht mehr als 50000 Pas, bevorzugt von nicht mehr als 10000 Pas, besonders bevorzugt von nicht mehr als 1000 Pas auf, gemessen mit einem Rotationsviskosimeter.

Zur gezielten Einstellung einer dynamischen Viskosität der Trägersubstanz kann diese eine Basisflüssigkeit und ein Verdickungsmittel umfassen. Die Basisflüssigkeit kann Wasser sein.

Das Verdickungsmittel kann ausgewählt sein aus einer Gruppe umfassend ein Biopolymer, wie etwa ein Polysaccharid, insbesondere Glykogen, Stärke, Pektin, Xanthan, Carrageenan, Guargummi, Gummi Arabicum, Cellulose oder ein Cellulosederivat, etwa Carboxymethylcellulose, Lignin, Chitin, Chitosan, Gelatine, Agar-Agar, ein Alginat, oder ein Polymer, wie etwa Polyvinylpyrrolidon, PolyDADMAC oder PolyAMPS, oder Glykol.

Anstelle einer durch ein Verdickungsmittel verdickte Basisflüssigkeit kann die Trägersubstanz auch ein Polymer oder/und ein Gel umfassen oder sein. Als Gel kann insbesondere ein sogenanntes Hydrogel verwendet werden.

Nachzutragen ist, dass alle in der vorliegenden Anmeldung gemachten Angaben zu physikalischen Parametern der Kalibrationssuspension, der darin aufgenommenen Festkörperpartikel und der Trägersubstanz bei Raumtemperatur und einer Normalatmosphäre von 1013 hPa ermittelt wurden bzw. zum Vergleich zu ermitteln sind. Wie eingangs ausgesagt wurde, kommt es für die Verwendbarkeit der Trägersubstanz darauf an, dass sie elektrisch leitfähig ist. Als besonders geeignet haben sich Trägersubstanzen erwiesen, deren elektrische Leitfähigkeit im Bereich von 0,01 bis 500 mS/cm, vorzugsweise im Bereich von 0,1 bis 200 mS/cm, besonders bevorzugt im Bereich von 1 bis 50 mS/cm liegt. Höchst bevorzugt kann die Leitfähigkeit sogar in einem Bereich von 1 bis 10 mS/cm liegen.

Bei allen Bereichsangaben in dieser Anmeldung sollen die angegebenen Grenzen stets als vom Bereich umfasst verstanden werden.

Diese elektrische Leitfähigkeit kann dadurch eingestellt werden, dass die Trägersubstanz bewegliche Ionen entgegengesetzter Ladungspolarität aufweist, etwa durch Lösung oder Schmelze von Salzen oder/und durch Protolyse von Säuren oder Basen und dergleichen. Die Lösung von Salzen ist dabei ihrer Schmelze aufgrund der hierfür notwendigen thermischen Randbedingungen bevorzugt.

Um ausschließen zu können, dass die Veränderung des lonengehalts der Trägersubstanz und damit der Kalibrationssuspension das Kalibrationsergebnis beeinflussen kann, ist gemäß einer vorteilhaften Weiterbildung der Verwendung der Kalibrationssuspension vorgesehen, dass die Konzentration von Ionen entgegengesetzter Ladungspolarität in der Trägersubstanz derart permittivitätsbezogen gesättigt ist, dass bei ansonsten gleichen Bedingungen eine Erhöhung der lonenkonzentration in der Trägersubstanz keine Erhöhung des Permittivitätswertes der Kalibrationssuspension bewirkt. Die Bedingungen sind dabei insbesondere Bedingungen zur Erfassung der Permittivität der Kalibrationssuspension.

Der Korngrößenbereich, der für in der Trägersubstanz aufzunehmenden bzw. aufgenommenen Festkörperpartikel anwendbar ist, reicht von 0,01 µm bis 500 µm. Bevorzugt werden Festkörperpartikel mit einer Korngröße im Bereich von 0,1 µm bis 200 µm in der Kalibrationssuspension verwendet. Dies kann auch vom jeweiligen Material der Festkörperpartikel abhängen. Besonders bevorzugt sind feine Festkörperpartikel mit einer Korngröße im Bereich von 1 µm bis 20 µm in der Trägersubstanz zur Bildung der Kalibrationssuspension aufgenommen. Die Korngröße kann dabei durch Siebung bestimmt werden. Sofern die Korngröße nicht durch Siebung ausreichend genau bestimmt werden kann, kann dies durch dynamische Lichtstreuung erfolgen. Beispielsweise kann Aluminiumpulver (Aluminiumpartikel) als Festkörperpartikel mit einer Korngröße von 100 bis 200 µm verwendet werden. Bevorzugt ist Graphitpulver (Graphitpartikel) als Festkörperpartikel in der Trägersubstanz aufgenommen, wobei für Graphitpartikel eine bevorzugte Korngröße im Bereich von 1 bis 20 µm verwendet wird. Auch Aktivkohlepartikel können als Festkörperpartikel in die Trägersubstanz zur Bildung einer Kalibrationssuspension aufgenommen sein. Bei Aktivkohlepartikeln reicht der bevorzugte Korngrößenbereich von 2 bis 12 µm.

Die Festkörperpartikel können aus einem Material mit einer elektrischen Leitfähigkeit im Bereich von 5 x 10⁻³ S/m bis 7 x 10⁷ S/m, bevorzugt im Bereich von 1 x 10⁴ S/m bis 4 x 10⁶ S/m gebildet sein.

Beispielsweise kann Tellur als Halbleiter als Festkörperpartikelmaterial verwendet werden. Es liegt in seiner elektrischen Leitfähigkeit von etwa 5 x 10⁻³ S/m am unteren Ende des genannten Bereichs. Am oberen Ende des genannten Leitfähigkeitsbereichs liegt beispielsweise Silber mit etwas weniger als 7 x 10⁷ S/m. Weitere mögliche und sogar bevorzugte Materialien für die Festkörperpartikel der Kalibrationssuspension sind Siliciumcarbid mit einer Leitfähigkeit von 50 S/m, Aluminium mit etwas weniger als 4 x 10⁶ S/m, Aktivkohle mit etwa 2 x 10⁴ S/m, Platin mit etwas mehr als 9 x 10⁶ S/m. Besonders bevorzugt wird Graphit als Festkörperpartikelmaterial verwendet. Graphit weist in seiner Leitfähigkeitsrichtung parallel zu seinen ebenen Schichten eine elektrische Leitfähigkeit von 3 x 10⁶ S/m auf.

Hinsichtlich seiner Art kann ein taugliches Festkörperpartikelmaterial ein Metall oder/und ein Halbmetall oder/und ein Halbleitermaterial oder/und ein leitfähiges organisches Material oder/und Graphit oder/und Aktivkohle umfassen oder aus wenigstens einem solchen Material bestehen. Die Kalibrationssuspension ist frei von Zellmaterial, wie Zellen, Zellmembranen und dergleichen.

Zur Vervollständigung der technischen Lehre betrifft die vorliegende Erfindung auch eine Kalibrationsanordnung, umfassend:
- eine von biologischen Bestandteilen freie Kalibrationssuspension, wie sie oben beschrieben und weitergebildet ist,
- einen impedanzspektroskopischen Biomassesensor, welcher zur Erfassung von Information über die Menge oder/und Größe von lebenden Zellen in einer Biomasse mittels eines elektrischen Feldes mit sich periodisch ändernder Feldrichtung ausgebildet ist und
- ein Datenverarbeitungsgerät, welches zur Verarbeitung von vom Biomassesensor betriebsgemäß abgegebenen Sensorsignalen ausgebildet ist und welches insbesondere zur Durchführung des oben beschriebenen und weitergebildeten Verfahrens ausgebildet ist.

Die vorliegende Erfindung wird nachfolgend anhand der beiliegenden Zeichnungen näher erläutert werden. Es stellt dar:
- Figur 1: einen grobschematischen Zusammenhang der Frequenzabhängigkeit der Permittivität einer Biomasse und
- Figur 2: die frequenzabhängige Permittivität der Biomasse grobschematisch im besonders interessierenden β-Dispersionsbereich.

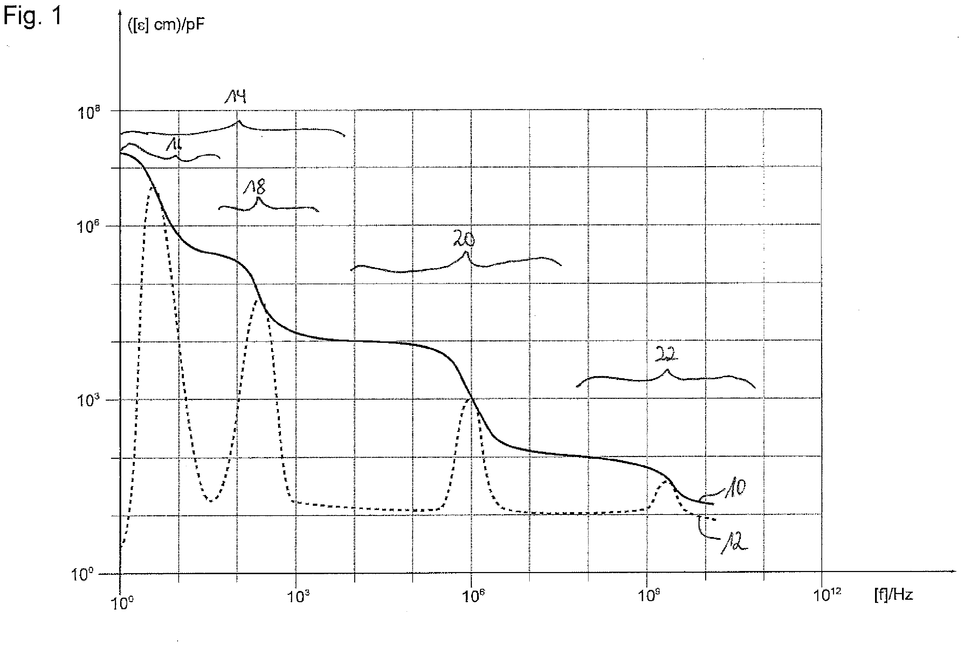

In Figur 1 ist ganz allgemein und lediglich grobschematisch die Frequenzabhängigkeit der Permittivität einer Biomasse, also beispielsweise einer biologische Zellen enthaltenden Flüssigkeit, dargestellt. Die durchgezogene Linie 10 zeigt den Realteil der Permittivität ε in Abhängigkeit von der Frequenz f, die gestrichelte Linie 12 zeigt den Imaginärteil der frequenzabhängigen Permittivität ε.

Die Graphen werden erhalten durch Anlegen eines elektrischen Feldes an eine Biomasse, dessen Feldrichtung periodisch verändert bzw. umgekehrt wird. Die Frequenz f der Feldrichtungsänderung in der Einheit Hz ist längs der Abszisse des Diagramms von Figur 1 aufgetragen. In Ordinatenrichtung ist die Permittivität ε in der Einheit pF/cm aufgetragen.

Wird die Frequenz f über einen großen Frequenzbereich hinweg allmählich erhöht, treten in der Biomasse aufgrund der darin enthaltenen Bestandteile unterschiedliche Polarisationseffekte auf, die zu einer Veränderung der Permittivität (und ebenso der Kapazität) zwischen den felderzeugenden Elektroden führen. Die einzelnen erkennbaren Stufen in der Permitivität-Frequenz-Kurve einer Biomasse werden nach H. P. Schwan als α-, β- und γ-Dispersionen bezeichnet. Die α-Dispersion (siehe α-Dispersionsbereich 14 in Figur 1) tritt bei Frequenzen im Millihertz- bis Kilohertzbereich auf. Die α-Dispersion wird nach derzeitigem Kenntnisstand durch die Bewegung und Anlagerung von Ladungsträgern auf der Oberfläche von Zellen hervorgerufen. Weiter wird die α-Dispersion durch aktive Zellmembraneffekte und aktive ionische Membrankanäle beeinflusst.

In Figur 1 weist der Frequenzbereich 14 der α-Dispersion zwei Teildispersionen a1, markiert durch den Bereich 16, und a2, markiert durch den Bereich 18, auf.

Der Frequenzbereich 20 der β-Dispersion liegt im Bereich von einigen Kilohertz bis etwa 100 MHz und beruht auf den kapazitiven Eigenschaften der Zellmembranen, etwa weil Fette und Proteine eine hochohmige Struktur bilden, und beruht weiter auf den intrazellulären Organellen und Membranstrukturen, wo im genannten Frequenzbereich eine Grenzflächenpolarisation auftritt.

Die γ-Dispersion tritt im Frequenzbereich 22 von etwa 0,1 bis 100 GHz auf. Sie wird hervorgerufen durch dipolare Mechanismen von polaren Medien wie Wasser und in Biomasse ebenfalls enthaltenen Proteinen. Es handelt sich dabei um eine Orientierungspolarisation, bewirkt durch große Moleküle mit Dipolmoment, die sich bei hohen Frequenzen ausrichten.

Der Realteil 10 der Permittivität ε weist bei den charakteristischen Frequenzen der jeweiligen Dispersion einen Wendepunkt auf, welcher üblicherweise zwischen zwei plateauähnlichen Frequenzbereichen liegt. Der Imaginärteil der Permittivität ε weist bei der jeweiligen charakteristischen Frequenz einer Dispersion ein lokales Maximum auf.

Während die γ-Dispersion im Frequenzbereich 22 aufgrund der dort auftretenden Polarisationseffekte zur Bestimmung des Wasseranteils von biologischen Objekten herangezogen werden kann, ist das β-Dispersionsgebiet im Frequenzbereich 20 für die Beurteilung von Biomasse von großem Interesse, da die Permittivität ε, insbesondere ihr Realteil, im β-Dispersionsbereich 20 Informationen über die Anzahl an in der Biomasse enthaltenen lebenden Zellen, über die Zellvitalität und über die Zellgröße liefert.

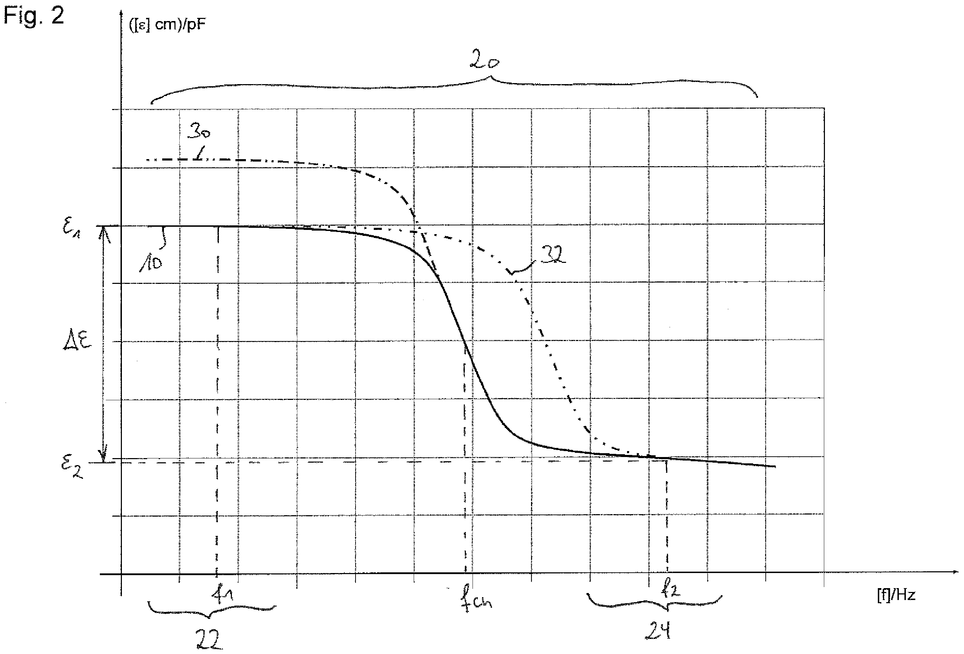

In Figur 2 ist rein qualitativ der Realteil einer frequenzabhängigen Permittivität ε einer Biomasse aufgetragen.

Dabei können qualitativ folgende Informationen aus der Kurve 10 des Realteils der Permittivität in Abhängigkeit von der Frequenz f erhalten werden. In Figur 2 ist qualitativ dargestellt, dass vor einer für das β-Dispersionsgebiet 20 charakteristischen Frequenz f_{Ch} ein Plateaugebiet 22 gelegen ist, in dem sich die Permittivität ε, verglichen mit dem Bereich um die charakteristische Frequenz f_{Ch}, mit der Frequenz nur wenig ändert, und dass nach der charakteristischen Frequenz f_{Ch} ein weiteres Plateaugebiet 24 gelegen ist, welches von dem Plateaugebiet 22 vor der charakteristischen Frequenz f_{Ch} verschieden ist und in welchem sich die Permittivität ε, wiederum verglichen mit dem Bereich um die charakteristische Frequenz f_{Ch} ebenfalls nicht stark mit der Frequenz ändert.

Erfasst man daher einen die Permittivität ε, genauer: deren Realteil, repräsentierenden Permittivitätswert ε₁ bei einer Frequenz fi im Plateaugebiet 22 und ebenso einen Permittivitätswert ε₂ bei einer Frequenz f₂ im Plateaugebiet 24, so lässt sich aus den beiden Frequenzen f₁ und f₂ ermittelten Permittivitätswerten ε₁ bzw. ε₂ ein Unterschiedswert Δε der beiden Permittivitätswerte ermitteln. Der Unterschiedswert Δε ist dabei ein Maß für die Anzahl an in der Biomasse enthaltenen lebenden Zellen. Die mit zwei Punkten und drei Strichen angedeutete alternative Permittivitätskurve 30 würde zu einem betragsmäßig größeren Δε bei den jeweiligen Messfrequenzen f₁ und f₂ führen, was den Rückschluss zulässt, dass die Biomasse, für welche die Permittivitätskurve 30 erhalten wurde, mehr lebende Zellen in gleichem Volumen aufweist als die der Permittivitätskurve 10 zu Grunde liegende Biomasse.

Der Vollständigkeit halber sei erwähnt, dass eine Veränderung der charakteristischen Frequenz f_{Ch} eine Änderung der Größe der Zellen oder ihrer Physiologie anzeigt. Eine Permittivitätskurve 32 mit zwei Punkten und einem Strich zeigt eine höhere charakteristische Frequenz in Figur 2.

Die Steigung der Permittivitätskurve im Punkt ihrer charakteristischen Frequenz f_{Ch} ist ein Maß für die Zellgrößenverteilung, wobei zunehmende Steigung eine stärker heterogene Zellgrößenverteilung anzeigt und wobei flacher werdende Verläufe der Permittivitätskurve 10 am Ort der charakteristischen Frequenz f_{Ch} stärker homogene Zellgrößenverteilungen bedeuten.

Ist für eine messtechnisch zu erfassende Biomasse das β-Dispersionsgebiet einigermaßen bekannt, sind die Frequenzen f₁ und f₂ als Messpunkte bekannt.

Die in dieser Anmeldung beschriebene Kalibrationssuspension liefert an den Messfrequenzen f₁ und f₂ definierte unterschiedliche Permittivitätswerte ε₁ bzw. ε₂, die zu einem ebenso definierten Unterschiedswert Δε führen.

Da die Kalibrationssuspension hinsichtlich der verwendeten Materialien: Trägersubstanz und Festkörperpartikel, und hinsichtlich der bei Herstellung der Suspension verwendeten Mengenverhältnisse bekannt ist, kann diese mit hoher Genauigkeit reproduziert werden.

Der Kalibrationssuspension kann somit durch entsprechende Messungen von Permittivitätswerten bei unterschiedlichen Frequenzen Bezugswerte zugeordnet werden, die aus einem Wertepaar, wie beispielsweise Messfrequenz und dabei gemessener Permittivitätswert, bestehen können. Durch mehrfache Wiederholung der Messungen können die Bezugswerte statistisch abgesichert sein. Auch die Streuung der Bezugswerte um einen Bezugswertemittelwert kann daher bekannt sein.

Somit kann für eine vorbestimmte Kalibrationssuspension bei vorbestimmten unterschiedlichen Messfrequenzen f₁ und f₂ aus den zugeordneten Bezugswerten der für die Kalibration heranzuziehende Bezugs-Unterschiedswert der Permittivitätswerte ermittelt werden. Bei der Durchführung eines Kalibrations-Messverfahrens unter Verwendung der zu kalibrierenden Biomassesensoren, jedoch unter Verwendung der definierten Kalibrationssuspension anstelle der Biomasse, wird somit ein Unterschiedswert der Permittivitätswerte sensorisch erfasst und mit dem Bezugs-Unterschiedswert verglichen Abhängig von dem Grad an Übereinstimmung zwischen den bei dem Kalibrationsverfahren erfassten Unterschiedswert und dem Bezugs-Unterschiedswert kann dann auf die Funktionstüchtigkeit der im Kalibrationsverfahren eingesetzten Biomassesensoren geschlossen werden, oder es können die Biomassesensoren entsprechend justiert werden, sodass der von ihnen unter definierten Kalibrationsbedingungen gelieferte Unterschiedswert der Permittivitätswerte der Kalibration mit dem entsprechenden Bezugs-Unterschiedswert übereinstimmt.

Das Kalibrationsverfahren, gegebenenfalls unter Anschluss des Justierverfahrens, kann in regelmäßigen Abständen durchgeführt werden oder kann jedes Mal vor der Durchführung einer sensorischen Erfassung einer Biomasse durchgeführt werden. Auch nach der sensorischen Erfassung einer Biomasse kann das Kalibrationsverfahren durchgeführt werden, um nachweisen zu können, dass sich die Funktionstüchtigkeit der Biomassesensoren während des Messverfahrens nicht verändert hat.

Zur Kalibration von Biomassesensoren können unterschiedliche Kalibrationssuspensionen bereitgestellt sein, die sich hinsichtlich Trägersubstanz oder/und Festkörperpartikel oder/und hinsichtlich der Menge an in der Trägersubstanz aufgenommenen Festkörperpartikel unterscheiden. Dadurch kann eine Kalibration unter Verwendung einer passend ausgewählten Kalibrationssuspension mit möglichst großer Ähnlichkeit zu dem zur sensorischen Erfassung der Biomasse durchgeführten Messverfahren ausgeführt werden. Somit kann die Aussagekraft des Kalibrationsverfahrens für ein mit denselben Biomassesensoren durchgeführtes Messverfahren vorteilhaft erhöht werden.

## Patentansprüche

1. Verfahren zur Kalibration von impedanzspektroskopischen Biomassesensoren, welche zur Erfassung von Information über die Menge oder/und Größe von lebenden Zellen in einer Biomasse mittels eines elektrischen Feldes mit sich periodisch ändernder Feldrichtung ausgebildet sind, umfassend die folgenden Schritte:
- Bereitstellen einer Kalibrationssuspension umfassend eine elektrisch leitfähige viskos-fließfähige oder elektrisch leitfähige viskoelastische Trägersubstanz und darin aufgenommene elektrisch leitfähige Festkörperpartikel oder/und Halbleiter-Festkörperpartikel,
- Erzeugen eines auf die Kalibrationssuspension einwirkenden elektrischen Feldes mit sich periodisch ändernder Feldrichtung,
- Erfassen von wenigstens je einem eine Permittivität der Kalibrationssuspension repräsentierenden Permittivitätswert bei wenigstens zwei elektrischen Feldern unterschiedlicher Frequenz der Feldrichtungsänderung, und
- Ermitteln eines einen Unterschied der erfassten Permittivitätswerte repräsentierenden Unterschiedswertes,
**dadurch gekennzeichnet, dass** das Verfahren den folgenden weiteren Schritt umfasst:
- Vergleichen des Unterschiedswertes mit einem der Kalibrationssuspension zugeordneten Bezugswert, und,
dass die Kalibrationssuspension frei von biologischen Bestandteilen ist.

2. Kalibrationsverfahren nach Anspruch 1,
**dadurch gekennzeichnet, dass** es vor oder während des Erzeugens des elektrischen Feldes ein Kontaktieren der Kalibrationssuspension mit wenigstens einem Abschnitt des Biomassesensors umfasst.

3. Kalibrationsverfahren nach Anspruch 2,
**dadurch gekennzeichnet, dass** das Kontaktieren der Kalibrationssuspension mit wenigstens einem Abschnitt des Biomassesensors ein Benetzen wenigstens einer Sensorelektrode, vorzugsweise aller Sensorelektroden, des Biomassesensors mit Kalibrationssuspension umfasst.

4. Kalibrationsverfahren nach einem der vorhergehenden Ansprüche,
**dadurch gekennzeichnet, dass** es einen Schritt der Homogenisierung der Kalibrationssuspension umfasst, beispielsweise durch Rühren oder/und Schütteln der Kalibrationssuspension.

5. Kalibrationsverfahren nach Anspruch 4,
**dadurch gekennzeichnet, dass** der Schritt der Homogenisierung der Kalibrationssuspension vor, bevorzugt unmittelbar vor der Erfassung der Permittivitätswerte ausgeführt wird.

6. Kalibrationsverfahren nach einem der vorhergehenden Ansprüche,
**dadurch gekennzeichnet, dass** die Permittivitätswerte bei Frequenzen von nicht weniger als 50 kHz, vorzugsweise nicht weniger als 110 kHz und besonders bevorzugt von nicht weniger als 250 kHz und von nicht mehr als 110 MHz, vorzugsweise von nicht mehr als 50 MHz, besonders bevorzugt von nicht mehr als 30 MHz, erfasst werden, die genannten Grenzwerte jeweils eingeschlossen.

7. Kalibrationsverfahren nach einem der vorhergehenden Ansprüche,
**dadurch gekennzeichnet, dass** es ein Justieren des Biomassesensors oder einer mit ihm signaltübertragungsmäßig gekoppelten Datenverarbeitungsvorrichtung nach Maßgabe des Ergebnisses des Vergleichs des Unterschiedswertes mit dem Bezugswert umfasst.

8. Verfahren zur impedanzspektroskopischen Erfassung von Information über die Menge oder/und Größe von lebenden Zellen in einer Biomasse, umfassend
- das Kalibrationsverfahren nach einem der vorhergehenden Ansprüche,
- dem nachfolgend: Reinigen des Biomassesensors,
- dem nachfolgend: impedanzspektroskopische Erfassung der Information über die Menge oder/und Größe von lebenden Zellen in der Biomasse mittels des Biomassesenors und einem von diesem erzeugten elektrischen Feld mit sich periodisch ändernder Feldrichtung,
- optional dem nachfolgend: das Kalibrationsverfahren nach einem der vorhergehenden Ansprüche.

9. Verwendung einer von biologischen Bestandteilen freien Suspension, umfassend eine elektrisch leitfähige viskos-fließfähige oder elektrisch leitfähige viskoelastische Trägersubstanz und darin aufgenommene elektrisch leitfähige Festkörperpartikel oder/und Halbleiter-Festkörperpartikel, zur Kalibration von impedanzspektroskopischen Biomassesensoren, welche zur Erfassung von Information über die Menge oder/und Größe von lebenden Zellen in einer Biomasse mittels eines elektrischen Feldes mit sich periodisch ändernder Feldrichtung ausgebildet sind.

10. Verwendung einer Suspension nach Anspruch 9,
**dadurch gekennzeichnet, dass** die Trägersubstanz eine dynamische Viskosität von nicht weniger als 1 Pas, bevorzugt von nicht weniger als 50 Pas, und von nicht mehr als 50000 Pas, bevorzugt von nicht mehr als 10000 Pas, besonders bevorzugt von nicht mehr als 1000 Pas aufweist, gemessen mit einem Rotationsviskosimeter.

11. Verwendung einer Suspension nach Anspruch 9 oder 10,
**dadurch gekennzeichnet, dass** die Trägersubstanz eine Basisflüssigkeit, etwa Wasser, und ein Verdickungsmittel umfasst.

12. Verwendung einer Suspension nach Anspruch 11,
**dadurch gekennzeichnet, dass** das Verdickungsmittel ein Biopolymer, wie etwa ein Polysaccharid, insbesondere Glykogen, Stärke, Pektin, Xanthan, Carrageenan, Guargummi, Gummi Arabicum, Cellulose oder ein Cellulosederivat, etwa Carboxymethylcellulose, Lignin, Chitin, Chitosan, Gelatine, Agar-Agar, ein Alginat, oder ein Polymer, wie etwa Polyvinylpyrrolidon, PolyDADMAC oder PolyAMPS, oder Glykol umfasst.

13. Verwendung einer Suspension nach einem der Ansprüche 9 bis 12,
**dadurch gekennzeichnet, dass** die Trägersubstanz ein Polymer oder/und ein Gel, insbesondere Hydrogel, umfasst oder ist.

14. Verwendung einer Suspension nach einem der Ansprüche 9 bis 13,
**dadurch gekennzeichnet, dass** die Trägersubstanz eine elektrische Leitfähigkeit im Bereich von 0,01 bis 500 mS/cm, vorzugsweise von 0,1 bis 200 mS/cm, besonders bevorzugt von 1 bis 50 mS/cm liegt.

15. Verwendung einer Suspension nach einem der Ansprüche 9 bis 14,
**dadurch gekennzeichnet, dass** die Trägersubstanz bewegliche Ionen entgegengesetzter Ladungspolarität aufweist, etwa durch Lösung oder Schmelze von Salzen oder/und durch Protolyse von Säuren oder Basen und dergleichen.

16. Verwendung einer Suspension nach Anspruch 15,
**dadurch gekennzeichnet, dass** die Konzentration von Ionen entgegengesetzter Ladungspolarität in der Trägersubstanz derart permittivitätsbezogen gesättigt ist, dass bei ansonsten gleichen Bedingungen eine Erhöhung der lonenkonzentration in der Trägersubstanz keine Erhöhung des Permittivitätswertes der Kalibrationssuspension bewirkt.

17. Verwendung einer Suspension nach einem der Ansprüche 9 bis 16,
**dadurch gekennzeichnet, dass** die Festkörperpartikel eine Korngröße im Bereich von 0,01 µm bis 500 µm, bevorzugt im Bereich von 0,1 µm bis 200 µm, besonders bevorzugt im Bereich von 1 µm bis 20 µm aufweisen, wobei die Korngröße durch Siebung zu bestimmen ist.

18. Verwendung einer Suspension nach einem der Ansprüche 9 bis 17,
**dadurch gekennzeichnet, dass** die Festkörperpartikel aus einem Material mit einer elektrischen Leitfähigkeit im Bereich von 5 x 10⁻³ S/m bis 7 x 10⁷ S/m, bevorzugt im Bereich von 1 x 10⁴ S/m bis 4 x 10⁶ S/m gebildet sind.

19. Verwendung einer Suspension nach einem der Ansprüche 9 bis 18,
**dadurch gekennzeichnet, dass** die Festkörperpartikel ein Metall oder/und ein Halbmetall oder/und ein Halbleitermaterial oder/und ein leitfähiges organisches Material oder/und Graphit oder/und Aktivkohle umfassen oder aus wenigstens einem solchen Material bestehen.

20. Kalibrationsanordnung, umfassend:
- einen impedanzspektroskopischen Biomassesensor, welcher zur Erfassung von Information über die Menge oder/und Größe von lebenden Zellen in einer Biomasse mittels eines elektrischen Feldes mit sich periodisch ändernder Feldrichtung ausgebildet ist,
- eine Kalibrationssuspension zur Kalibration des impedanzspektroskopischen Biomassesensors , wobei die Kalibrationssuspension eine elektrisch leitfähige viskos-fließfähige oder elektrisch leitfähige viskoelastische Trägersubstanz sowie darin aufgenommene elektrisch leitfähige Festkörperpartikel oder/ und Halbleiter-Festkörperpartikel umfasst, und
- ein Datenverarbeitungsgerät, welches zur Verarbeitung von vom Biomassesensor betriebsgemäß abgegebenen Sensorsignalen ausgebildet ist,
**dadurch gekennzeichnet, dass** die Kalibrationssuspension von biologischen Bestandteilen frei ist

## Claims

1. A method for calibrating impedance-spectroscopic biomass sensors that are embodied to detect information regarding the quantity and/or size of living cells in a biomass by means of an electric field having a periodically changing field direction, encompassing the following steps:
- furnishing a calibration suspension encompassing an electrically conductive viscously flowable or electrically conductive viscoelastic carrier substance as well as electrically conductive solid particles and/or solid semiconductor particles received therein,
- generating an electric field, having a periodically changing field direction, which acts on the calibration suspension;
- detecting at least one permittivity value, respectively representing a permittivity of the calibration suspension, in a context of at least two electric fields having different field direction change frequencies; and
- ascertaining a difference value representing a difference between the detected permittivity values;
**characterized in that** the method comprises the following additional step:
- comparing the difference value with a reference value associated with the calibration suspension, and **in that** the calibration suspension is free from biological components.

2. The calibration method according to Claim 1,
**characterized in that** it encompasses, before or during generation of the electric field, contact between the calibration suspension and at least a portion of the biomass sensor.

3. The calibration method according to Claim 2,
**characterized in that** contact between the calibration suspension and at least a portion of the biomass sensor encompasses wetting at least one sensor electrode, preferably all sensor electrodes, of the biomass sensor with calibration suspension.

4. The calibration method according to one of the preceding claims,
**characterized in that** it encompasses a step of homogenizing the calibration suspension, for example by stirring and/or shaking the calibration suspension.

5. The calibration method according to Claim 4,
**characterized in that** the homogenizing step is performed before, preferably immediately before, detection of the permittivity values.

6. The calibration method according to one of the preceding claims,
**characterized in that** the permittivity values are detected at frequencies no lower than 50 kHz, preferably no lower than 110 kHz, and particularly preferably no lower than 250 kHz, and no higher than 110 MHz, preferably no higher than 50 MHz, particularly preferably no higher than 30 MHz, in each case including the aforementioned limit values.

7. The calibration method according to one of the preceding claims,
**characterized in that** it encompasses adjusting the biomass sensor, or a data processing apparatus coupled to it in signal-transferring fashion, in accordance with the result of the comparison of the difference value with the reference value.

8. A method for impedance-spectroscopic detection of information regarding the quantity and/or size of living cells in a biomass, encompassing:
- the calibration method according to one of the preceding claims;
- subsequently thereto: cleaning of the biomass sensor;
- subsequently thereto: impedance-spectroscopic detection of information regarding the quantity and/or size of living cells in the biomass, by means of the biomass sensor and an electric field, generated thereby, having a periodically changing field direction;
- optionally subsequently thereto: the calibration method according to one of the preceding claims.

9. Use of a suspension that is free of biological components, encompassing an electrically conductive viscously flowable or electrically conductive viscoelastic carrier substance as well as electrically conductive solid particles and/or solid semiconductor particles received therein, to calibrate impedance-spectroscopic biomass sensors that are embodied to detect information regarding the quantity and/or size of living cells in a biomass by means of an electric field having a periodically changing field direction.

10. The use of a suspension according to Claim 9,
**characterized in that** the carrier substance has a dynamic viscosity of no less than 1 Pas, preferably no less than 50 Pas, and of no more than 50,000 Pas, preferably no more than 10,000 Pas, particularly preferably no more than 1000 Pas, measured with a rotational viscometer.

11. The use of a suspension according to Claim 9 or 10,
**characterized in that** the carrier substance encompasses a base liquid, for example water, and a thickening agent.

12. The use of a suspension according to Claim 11,
**characterized in that** the thickening agent encompasses a biopolymer, for example a polysaccharide, in particular glycogen, starch, pectin, xanthan, carrageenan, guar gum, gum arabic, cellulose or a cellulose derivative, for example carboxymethyl cellulose, lignin, chitin, chitosan, gelatin, agar-agar, an alginate, or a polymer, for example polyvinylpyrrolidone, polyDADMAC or polyAMPS, or glycol.

13. The use of a suspension according to one of Claims 9 to 12, **characterized in that** the carrier substance encompasses or is a polymer and/or a gel, in particular a hydrogel.

14. The use of a suspension according to one of Claims 9 to 13,
**characterized in that** the carrier substance has an electrical conductivity in the range from 0.01 to 500 mS/cm, preferably from 0.1 to 200 mS/cm, particularly preferably from 1 to 50 mS/cm.

15. The use of a suspension according to one of Claims 9 to 14,
**characterized in that** the carrier substance comprises movable ions of opposite charge polarity, for example due to the dissolution or melting of salts and/or by protolysis of acids or bases.

16. The use of a suspension according to Claim 15,
**characterized in that** the concentration of ions of opposite charge polarity in the carrier substance is saturated in permittivity-related fashion in such a way that under otherwise identical conditions, an elevation of the ion concentration in the carrier substance does not cause any elevation of the permittivity value of the calibration suspension.

17. The use of a suspension according to one of Claims 9 to 16,
**characterized in that** the solid particles have a particle size in the range from 0.01 µm to 500 µm, preferably in the range from 0.1 µm to 200 µm, particularly preferably in the range from 1 µm to 20 µm, the particle size being determined by sieving.

18. The use of a suspension according to one of Claims 9 to 17,
**characterized in that** the solid particles are constituted from a material having an electrical conductivity in the range from 5 x 10⁻³ S/m to 7 x 10⁷ S/m, preferably in the range from 1 x 10⁴ S/m to 4 x 10⁶ S/m.

19. The use of a suspension according to one of Claims 9 to 18,
**characterized in that** the solid particles encompass a metal and/or a semi-metal and/or a semiconductor material and/or a conductive organic material and/or graphite and/or activated carbon, or are made of at least one such material.

20. A calibration arrangement, encompassing:
- an impedance-spectroscopic biomass sensor that is embodied to detect information regarding the quantity or/and size of living cells in a biomass by means of an electric field having a periodically changing field direction,
- a calibration suspension for calibrating the impedance-spectroscopic biomass sensor, wherein the calibration suspension encompasses an electrically conductive viscously flowable or electrically conductive viscoelastic carrier substance as well as electrically conductive solid particles and/or solid semiconductor particles received therein, and
- a data processing device that is embodied to process sensor signals delivered operationally by the biomass sensor,
**characterized in that** the calibration suspension is free from biological components.

## Revendications

1. Procédé de calibrage de capteurs de biomasse spectroscopiques à impédance, qui sont adaptés pour acquérir des informations sur la quantité et/ou la taille de cellules vivantes dans une biomasse au moyen d'un champ électrique dont la direction de champs change périodiquement, comprenant les étapes suivantes :
- fournir une suspension de calibrage comprenant une substance de support électriquement conductrice visco-écoulable ou électriquement conductrice viscoélastique et des particules solides électriquement conductrices et/ou des particules solides semi-conductrices qui y sont incorporées,
- générer un champ électrique agissant sur la suspension de calibrage avec changement périodique de la direction de champ,
- détecter au moins une valeur de permittivité représentant une permittivité de la suspension de calibrage pour au moins deux champs électriques de fréquence différente du changement de direction du champ, et
- déterminer une valeur de différence représentant une différence dans les valeurs de permittivité détectées,
**caractérisé en ce que** le procédé comprend en outre l'étape suivante :
- comparer la valeur de différence avec une valeur de référence associée à la suspension de calibrage, et **en ce que** la suspension de calibrage est exempte de constituants biologiques.

2. Procédé de calibrage selon la revendication 1,
**caractérisé en ce qu'il** comprend la mise en contact de la suspension de calibrage avec au moins une section du capteur de biomasse avant ou pendant la génération du champ électrique.

3. Procédé de calibrage selon la revendication 2,
**caractérisé en ce que** la mise en contact de la suspension de calibrage avec au moins une section du capteur de biomasse comprend le mouillage d'au moins une électrode de capteur, de préférence de toutes les électrodes de capteur, du capteur de biomasse avec la suspension de calibrage.

4. Procédé de calibrage selon l'une des revendications précédentes,
**caractérisé en ce qu'il** comprend une étape d'homogénéisation de la suspension de calibrage, par exemple en agitant et/ou en secouant la suspension de calibrage.

5. Procédé de calibrage selon la revendication 4,
**caractérisé en ce que** l'étape d'homogénéisation de la suspension de calibrage est effectuée avant, de préférence immédiatement avant, la détection des valeurs de permittivité.

6. Procédé de calibrage selon l'une quelconque des revendications précédentes,
**caractérisé en ce que** les valeurs de permittivité sont détectées à des fréquences non inférieures à 50 kHz, de préférence non inférieures à 110 kHz et particulièrement de préférence non inférieures à 250 kHz et non supérieures à 110 MHz, de préférence non supérieures à 50 MHz, particulièrement de préférence non supérieures à 30 MHz, incluant respectivement lesdites valeurs limites.

7. Procédé de calibrage selon l'une des revendications précédentes,
**caractérisé en ce qu'il** comprend l'ajustement du capteur de biomasse ou d'un dispositif de traitement de données qui lui est couplé par transmission de signaux, en fonction du résultat de la comparaison de la valeur de différence avec la valeur de référence.

8. Procédé d'acquisition par spectroscopie d'impédance d'informations sur la quantité et/ou la taille de cellules vivantes dans une biomasse, comprenant :
- le procédé de calibrage selon l'une des revendications précédentes,
- y suivant : nettoyage du capteur de biomasse,
- y suivant : acquisition par spectroscopie d'impédance d'informations sur la quantité et/ou la taille de cellules vivantes dans la biomasse au moyen du capteur de biomasse et d'un champ électrique qui est généré par celui-ci et dont la direction de champ change périodiquement et,
- éventuellement y suivant : le procédé de calibrage selon l'une des revendications précédentes.

9. Utilisation d'une suspension exempte de constituants biologiques, comprenant une substance de support électriquement conductrice visco-écoulable ou électriquement conductrice viscoélastique et des particules solides électriquement conductrices et/ou des particules solides semi-conductrices qui y sont incorporées, pour le calibrage de capteurs de biomasse spectroscopiques à impédance qui sont adaptés pour détecter des informations sur la quantité et/ou la taille de cellules vivantes dans une biomasse au moyen d'un champ électrique dont la direction de champ change périodiquement.

10. Utilisation d'une suspension selon la revendication 9,
**caractérisée en ce que** la substance de support a une viscosité dynamique non inférieure à 1 Pas, de préférence non inférieure à 50 Pas, et non supérieure à 50.000 Pas, de préférence non supérieure à 10.000 Pas, particulièrement de préférence non supérieure à 1.000 Pas, mesurée avec un viscosimètre rotatif.

11. Utilisation d'une suspension selon les revendications 9 ou 10,
**caractérisée en ce que** la substance de support comprend un liquide de base, tel que de l'eau, et un agent épaississant.

12. Utilisation d'une suspension selon la revendication 11,
**caractérisée en ce que** l'agent épaississant comprend un biopolymère, tel qu'un polysaccharide, en particulier le glycogène, l'amidon, la pectine, la xanthane, la carraghénane, la gomme de guar, la gomme arabique, la cellulose ou un dérivé de la cellulose, tel que la carboxyméthylcellulose, la lignine, la chitine, le chitosane, la gélatine, l'agar-agar, un alginate, ou un polymère, tel que la polyvinylpyrrolidone, le PolyDADMAC ou le PolyAMPS, ou le glycol.

13. Utilisation d'une suspension selon l'une des revendications 9 à 12,
**caractérisée en ce que** la substance de support comprend ou est un polymère et/ou un gel, en particulier un hydrogel.

14. Utilisation d'une suspension selon l'une des revendications 9 à 13,
**caractérisée en ce que** la substance de support présente une conductivité électrique dans la gamme de 0,01 à 500 mS/cm, de préférence de 0,1 à 200 mS/cm, de manière particulièrement préférée de 1 à 50 mS/cm.

15. Utilisation d'une suspension selon l'une des revendications 9 à 14,
**caractérisée en ce que** la substance de support présente des ions mobiles de polarité de charge opposée, par exemple par solution ou fusion de sels et/ou par protolyse d'acides ou de bases et similaires.

16. Utilisation d'une suspension selon la revendication 15,
**caractérisée en ce que** la concentration d'ions de polarité de charge opposée dans la substance de support est saturée en ce qui concerne la permittivité de telle manière que, dans des conditions par ailleurs identiques, une augmentation de la concentration d'ions dans la substance de support ne provoque pas une augmentation de la valeur de permittivité de la suspension de calibrage.

17. Utilisation d'une suspension selon l'une des revendications 9 à 16,
**caractérisée en ce que** les particules solides ont une taille de particules dans la gamme de 0,01 µm à 500 µm, de préférence dans la gamme de 0,1 µm à 200 µm, de manière particulièrement préférée dans la gamme de 1 µm à 20 µm, la taille des particules étant déterminée par tamisage.

18. Utilisation d'une suspension selon l'une des revendications 9 à 17,
**caractérisée en ce que** les particules solides sont formées d'un matériau ayant une conductivité électrique dans la gamme de 5 x 10⁻³ S/m à 7 x 10⁷ S/m, de préférence dans la gamme de 1 x 10⁴ S/m à 4 x 10⁶ S/m.

19. Utilisation d'une suspension selon l'une des revendications 9 à 18,
**caractérisée en ce que** les particules solides comprennent un métal et/ou un semi-métal et/ou un matériau semi-conducteur et/ou un matériau organique conducteur et/ou du graphite et/ou du charbon actif ou consistent en au moins un tel matériau.

20. Agencement de calibrage comprenant :
- un capteur de biomasse spectroscopique à impédance qui est adapté pour détecter des informations sur la quantité et/ou la taille de cellules vivantes dans une biomasse au moyen d'un champ électrique dont la direction de champ change périodiquement,
- une suspension de calibrage pour le calibrage du capteur de biomasse spectroscopique à impédance, la suspension de calibrage comprenant une substance de support électriquement conductrice visco-écoulable ou électriquement conductrice viscoélastique et des particules solides électriquement conductrices et/ou des particules solides semi-conductrices qui y sont incorporées, et
- un dispositif de traitement des données qui est adapté pour traiter les signaux émis par le capteur de biomasse en fonction du fonctionnement,
**caractérisé en ce que** la suspension de calibrage est exempte de constituants biologiques.
